# DEMANDE DE BREVET EUROPEEN

(11) **EP 1 038 520 A1**
(43) Date de publication de la demande: **27.09.2000**
(21) Numéro de dépôt: 99480012.6
(22) Date de dépôt: 16.03.1999
(51) Int. Cl.: A61K 7/48, A61K 31/215

(54) **Cosmetiques contenant du meclofenoxate**

(71) Demandeur: Chabrier, Pierre-Marie, 06400 Cannes (FR)
(72) Inventeur: Chabrier, Pierre-Marie, 06400 Cannes (FR)

(57) **Abrégé**

L'invention concerne l'utilisation de p-chlorophénoxyacétate de diméthylaminoéthyle dans des préparations cosmétiques et/ou dermatologiques pour appliquer à la peau ses qualités stimulantes sur les tissus neurologiques,(en raison de leur origine embryologique commune) : activation de la microcirculation, de la consommation d'O2 et du métabolisme, des systèmes antiradicaux libres, et réduction des dépôts de lipofuchsine. L'effet est une amélioration de l'aspect de la peau (rides, lissage, acnée, "peau d'orange", vergetures, cicatrices, pigmentations), une augmentation de la fermeté et de l'élasticité, une stimulation de la repousse et de la qualité des cheveux et des ongles.

## Description

Les effets du vieillissement cutané sont biens connus, avec altération à tous les niveaux, dermiques et épidermiques, de la multiplication cellulaire, de la qualité des tissus de soutien, de la microcirculation, et accumulation de dépots pigmentaires. Ces phénomènes naturels peuvent être majorés par l'exposition solaire( héliodermie ), le tabac ou d'autres facteurs pathologiques ou oncogènes.

On trouve toujours dans la génèse de ces troubles le rôle des radicaux libres de l'oxygène ( ou formes réactives de l'oxygène - F RO )destructeurs de molécules biologiques, dont la formation est la conséquence inévitable du fait de respirer de l'oxygène. Inévitable mais nocive bien qu'à l'état normal, la présence de molécules antioxydantes assure un état de réduction permanent.

Les découvertes modernes de neuro-dermatologie montrent qu'il existe dans tous les processus d'altérations cutanées par vieillissement ou pathologies des facteurs incontournables: les neuromédiateurs, auxquels sont sensibles les kératinocytes; il y a communication entre la peau et le cerveau du fait de leur origine embryologique commune, l'ectoblaste.

Les kératinocytes possèdent des récepteurs à ces molécules synthétisées par les neurones et en produisent également. Ils sont donc au centre d'interactions complexes avec le système nerveux central.

On suppose même que ces cellules communiquent entre elles, et de nombreux neuromédiateurs sont retrouvés dans la peau: substance P,somatostatine, Cgrp, Vip, histamine, sérotonine...Leur concentration varie selon certaines pathologies, certains réduisant le phénomène allergique en inhibant la présentation antigénique, d'autres favorisant la prolifération ou l'inhibition des kératinocytes.

Bien sûr leur action se retrouve au niveau de la régulation des radicaux libres et de la microcirculation.

Les différents traitements cosmétiques et / ou dermatologiques proposés pour tenter de prévenir ou réparer les problèmes décrits ci-dessus apportent des hormones, des antiradicaux libres, des oligo éléments, des vitamines, des macromolécules du tissu conjonctif, des acides gras essentiels, des extraits biologiques placentaires ou tissulaires; toutes ont une activité partielle et insuffisante.

Il convient de faire une place à part à l'acide rétinoïque qui a fait l'objet de publications scientifiques montrant son intérêt dans le traitement du vieillissement photo induit; soulignons l'activité des alpha hydroxyacides dans cette indication, avec des effets secondaires moins marqués.

Le traitement idéal doit donc être étiologique, c'est à dire agir sur les neuromédiateurs et leurs conséquences antiradicalaires et microcirculatoires, ainsi qu'optimiser la consommation d'oxygène de façon à limiter la formation des FRO gage d'une sauvegarde maximale des cellules et de leur contenu.

Il nous a semblé logique et opportun d'utiliser un actif connu pour ses qualités neurologiques et cérébrale:

Le P-CHLOROPHENOXYACETATE DE DIMETHYLAMINOETHYLE OU MECLOFENOXATE en raison de ses actions: une nette activation métabolique avec augmentation de la différence artério-veineuse du glucose et baisse du rapport lactate / pyruvate dans le sang veineux, une augmentation de la captation et de l'utilisation de l'oxygène, une nette augmentation du débit sanguin cérébral, une réduction de l'accumulation de lipofuchsine dans différents tissus, ces dépôts pigmentaires étant composés de produits de dégradation résultant de la peroxydation des lipides intra ou extra cellulaires et membranaires, une augmentation de la durée et l'espérance de vie chez certaines espèces.

Le Méclofénoxate stimule donc toute la chaine métabolique y compris les enzymes anti-oxydantes.

Nous avons retrouvé ces qualités pour des traitements cosmétiques et / ou dermatologiques dans des indications très larges, le mode d'action du Méclofénoxate pouvant intervenir dans toutes pathologies de la peau et phanères.

L'activité cutanée a été mise en évidence par tests cliniques chez des volontaires présentant des signes d'héliodermie; ils ont appliqué matin et soir pendant 90 jours une crème contenant 2% de Méclofénoxate sur une moitié du visage, tandis que l'excipient seul était utilisé de l'autre côté.

Nous avons constaté une nette amélioration des stigmates de l'héliodermie. Du côté traité on observe une atténuation des ridules, une amélioration de la texture cutanée et de l'épaisseur du derme, un lissage de l'épiderme et une atténuation des dépôts pigmentaires, voire une disparition des teints " brouillés". Aucun cas d'intolérance n'a été signalé.

Nous avons également effectué des biopsies sur deux patients volontaires avant et après 3 mois de traitement sur l'avant bras; l'examen anatomo pathologique a montré une amélioration de la trophicité cutanée, une diminution des lésions de dégénérescence des faisceaux de fibres élastiques, surtout au niveau du derme papillaire, une homogénéisation de la coloration des fibres de collagène.

Nous avons testé l'activité du Méclofénoxate associé à des kératolytiques, par observation clinique; comme on pouvait s'y attendre, nous avons noté une potentialisation permettant d'utiliser des doses de kératolytiques moindres que lorsqu'ils sont employés seuls, réduisant d'autant leurs effets secondaires irritants, surtout pour la Trétinoïne.

Le ou les agents kératolytiques utilisés selon l'invention peuvent être choisis parmi les alpha hydroxyacides (acide glycolique, mandélique, lactique, tartrique, citrique) ,les béta hydroxyacides et leurs dérivés, les rétinoïdes ( acide rétinoïque ou rétinol) et leurs dérivés, le peroxyde de benzoyle, ainsi que les sels de ces différents composés.

Le meilleur rapport efficacité tolérance a été obtenu par l'association du Méclofénoxate avec des alpha hydroxyacides, préférentiellement du lactate d'ammonium à la concentration de 5% à 15% en poids par rapport au poids total de la composition, et de l'acide salicylique à la concentration de 0,5% à 5% en poids par rapport au poids total de la composition.

L'activité capillaire a été mise en évidence par observation clinique sur des volontaires présentant différents niveaux de calvitie ou d'états séborrhéiques.

Nous avons utilisé des lotions à base de propylène glycol ou de teintures mères pour leur apport en oligo-éléments et actifs circulatoires, à des concentrations de 8 à 10% de méclofénoxate.

Les résultats ont été constamment positifs, avec repousses ou arrêt de la chute, augmentation de volume du cheveu, et dans tous les cas régulation de l'hyperséborrhée; bien sûr, l'effet reste limité et insuffisant en cas d'alopécie hipocratique. Les résultats sont de meilleur qualité avec les lotions à base de teintures méres ( Hedera Helix, Urtica Dioica, Equisetum Arvense). Aucun cas d'intolérance n'a été signalé.

Le Méclofénoxate peut donc être employé dans les produits cosmétiques et / ou dermatologiques de toutes sortes, pour la prévention et le traitement de la peau et des phanères, dès lors qu'il s'agit de stimuler le métabolisme cellulaire, la micro circulation, les systèmes antiradicaux libres, et réguler la mélanogénèse et la séborrhée. Leur application améliore l'aspect de la peau ( visage, mains, corps ) avec effet de lissage, tonique, antirides, restructure l'épiderme, restaure l'élasticité et la fermeté de la peau, traite la peau grasse et acnéique, accélère les processus de cicatrisation et prévient l'asphixie de la peau, régule la mélanogénèse en effacant les taches pigmentaires et éclaircissant le teint, gomme l'aspect "peau d'orange" de la cellulite et les vergetures.

L'application sur le cuir chevelu prévient la chute des cheveux,stimule leur repousse, leur croissance et leur aspect ( brillance, vigueur, souplesse), et traite l'hyper séborrhée et les états pelliculaires. Les mêmes effets sont obtenus au niveau des ongles.La concentration en Méclofénoxate varie dans les indications cutanées de 1 % à 5% en poids par rapport au poids total de la composition.

La concentration en Méclofénoxate varie dans les indications phanériennes de 5% à 20% en poids par rapport au poids total de la composition, préférentiellementde 5% à 10% pour les cheveux, et de 5% à 15% pour les ongles.

Le Méclofénoxate peut être incorporé dans toute forme galénique employée en cosmétique ou dermatologie, à savoir les émulsions H/E et E/H, laits, lotions, gels, pommades, huiles pour le corps, lotions capillaires, shampoings, savons, sticks et crayons, vernis, sprays, ainsi que dans des vecteurs comme les liposomes, les chylomicrons, les macro-, micro-, et nanocapsules, ou absorbé sur des polymères organiques poudreux, les talcs, bentonites et autres supports minéraux ou magnétiques.

Le Méclofénoxare peut être combiné avec tout autre ingrédient habituellement utilisé en cosmétique et dermatologie: lipides d'extraction et / ou de synthèse, polymères gélifiant et viscosant, tensioactifs et émulsifiant, principes actifs hydro ou lipo solubles, extraits de plantes, extraits biologiques, extraits marins, eaux thermales et / ou minérales.

## Revendications

1. Compositions cosmétiques et/ou dermatologiques possédant une activité stimulante et régénérante sur la peau et les phanères, caractérisées en ce qu'elles contiennent un ou des actifs kératolytiques, associé(s) à du Méclofénoxate on raison de ses qualités métaboliques, dans le but d'aumenter leur potentiel de réparation cutanée, tout on diminuant leur concentration et leurs effets irritants.

2. Compositions cosmétiques et/ou dermatologiques selon la revendication 1, caractérisées en ce qu'elles contiennent un ou des agents kératolytiques choisis parmi les alpha hydroxyacides (acide glycolique, mandélique, lactique, tartrique, citrique), les bêta hydroxyacides et leurs dérivés, les rétinoïdes et leurs dérivés, le peroxyde de benzoyle, ainsi que tes sels de ces différents composés.

3. Compositions cosmétiques et/ou dermatologiques selon l'une quelconque des revendications 1 et 2, caractérisées on ce que le ou les agents kératolytiques seront préférentiellement du lactate d'ammonium à la concentration de 5% à 15%, de l'acide salicylique à la concentration de 0,5% à 5%, ces concentrations étant exprimées en poids par rapport au poids total de la composition.

4. Compositions cosmétiques et/ou dermatologiques selon l'une quelconque des revendications 1 à 3, caractérisées on ce qu'elles seront utilisées on lotions capillaires associées préférentiellement à des extraits végétaux à base de teinture mère.

5. Compositions cosmétiques et/ou dermatologiques selon l'une quelconque des revendications 1 à 4, caractérisées en ce que la concentration en Méclofénoxate associé aux agents kératolytiques va de1% à 20% en poids par rapport au poids total de la composition.

6. Compositions cosmétiques et/ou dermatologiques selon l'une quelconque des revendications 1 à 5, caractérisées en ce que dans les indications cutanées la concentration en Méclofénoxate associé va préférentiellement de1% à 5% en poids par rapport au poids total de la composition.

7. Compositions cosmétiques et/ou dermatologiques selon l'une quelconque des revendications 1 à 6, caractérisées en ce que dans les indications capillaires la concentration en Méclofénoxate associé va préférentiellement de 5% à 10% en poids par rapport au poids total de la composition, et de 5% à 15% en poids par rapport au poids total de la composition pour les ongles.

8. Compositions cosmétiques et/ou dermatologiques selon l'une quelconque des revendications 1 à 7, caractérisées en ce qu'elles pourront être sous toutes formes galéniques employées en cosmétologie et dermatologie, à savoir les émulsions H/E et E/H, laits, lotions, gels, pommades, huiles pour le corps, lotions capillaires, shampoings, savons, sticks et crayons, vernis, sprays, ainsi que dans des vecteurs comme les liposomes, les chylomicrons, les macro-, micro-, nanocapsules, ou absorbées sur des polymères organiques poudreux, les talcs, bentonites et autres supports minéraux ou magnétiques.

9. Compositions cosmétiques et/ou dermatologiques selon l'une quelconque des revendications 1 à 8, caractérisées en ce qu'elles sont combinées avec tout autre ingrédient habituellement utilisé en cosmétologie et dermatologie: lipides d'extraction et/ou de synthèse, polymères gélifiant et viscosant, tensioactifs et émulsifiant, principes actifs hydro- ou liposolubles, extraits de plantes, extraits biologiques, extraits marins, eaux thermales et/ou minérales.

10. Compositions cosmétiques et/ou dermatologiques selon l'une quelconque des revendications 1 à 9, caractérisées en ce qu'elles sont utilisées pour tous les soins de la peau et des phanères, y compris le traitement raffermissant, tonique, antirides, l'effet de lissage, pour le traitement et la prévention des troubles de la pigmentation, l'éclaircissement des peaux foncéès, la stimulation de la cicatrisation et de la circulation, le t'alternent des peaux grasses et acnéiques, de l'aspect "peau d'orange" de la cellulite, et des vergetures, pour la stimulation de la repousse, de la croissance et de la qualité des cheveux et des ongles, pour la prévention des chutes de cheveux et le traitement des états pelliculaires.
